# EUROPEAN PATENT SPECIFICATION

(11) **EP 0 629 128 B1**
(45) Date of publication and mention of the grant of the patent: **06.09.1995**
(21) Application number: 91903914.9
(22) Date of filing: 11.02.1991
(51) Int. Cl.: A61K 9/127, A61K 7/00

(54) **AQUEOUS PHOSPHOLIPID VESICLE DISPERSION, PROCESS FOR ITS MANUFACTURE AND USE THEREOF**
WÄSSRIGE PHOSPHOLIPIDVESIKELDISPERSION, VERFAHREN ZU DEREN HERSTELLUNG SOWIE DEREN VERWENDUNG
DISPERSION DE VESICULES PHOSPHOLIPIDES AQUEUSE, PROCEDE DE PREPARATION ET UTILISATION

(30) Priority: 23.02.1990 DE 4005711
(43) Date of publication of application: 21.12.1994
(73) Proprietor: A. Nattermann & Cie. GmbH, D-50829 Köln (DE)
(72) Inventor: LAUTENSCHLÄGER, Hans, Heiner, D-5024 Pulheim 3 (DE); RÖDING, Joachim, D-5000 Köln 1 (DE); GHYCZY, Miklos, D-5000 Köln 41 (DE)
(74) Representative: Patentanwälte Hauck, Graalfs, Wehnert, Döring, Siemons
(86) International application number: EP9100267
(87) International publication number: WO9112794

(56) References cited:
- EP-A- 0 088 046
- EP-A- 0 095 591
- EP-A- 0 299 937
- WO-A-90/12565
- FR-A- 2 455 458
- SOFW, volume 115, no. 18, 21 November 1989, (Augsburg, DE), H. Lautenschläger: "Kosmetische Formulierungen mit Liposomen und Phospholipiden-Umfeld und Zusammenhänge", pages 662-663

## Description

The subject of the invention is an aqueous dispersion of phospholipid vesicles, a process for its manufacture and its use in pharmaceutical or cosmetic preparations.

Liposomes are vesicles and can possess structures of very different types. Depending on the method of preparation it is possible to distinguish unilamellar, oligolamellar, multilamellar or fused bodies with a membrane structure and a diameter of about 15 to 3500 nm.

In generally accepted speech liposomes are prepared from natural, semisynthetic or synthetic phospholipids, whereby the principal component is usually phosphatidylcholine. Other components are typically phosphatidylethanolamine, phosphatidylinositol and phosphatidic acid. A distinction is made between unsaturated (natural), partially hydrogenated and hydrogenated phospholipids according to their fatty acid composition. The subject is reviewed in H.P. Fiedler, Lexikon der Hilfsstoffe fur Pharmazie, Kosmetik und angrenzende Gebiete, Verlag Editio Cantor, Aulendorf 1989, pp 744-746.

In their article Lautenschläger et al. have emphasized the importance of liposomes from soy phospholipids in cosmetics (Seifen-Öle-Fette-Wachse 114 (1988), 531-534), while Skoza and Papahadjopoulos have provided a detailed description of the preparation of specific phospholipid vesicles (Ann. Rev. Biophys. Bioeng. 1980, 9, 467-476).

However, the present invention is not suggested even by a combination of the above mentioned publications.

In a manner similar to biological cells liposomes can take up water-soluble substances in the internal vesicular volumes and amphiphilic and lipophilic substances in the membranes (loaded liposomes). Experience has shown that loading of the membranes is of more interest for the application of liposomes in cosmetics and pharmaceuticals than loading of the internal vesicular volume, since in the former case the loaded substance remains largely associated with the membranes when the liposomes are mixed with other components of the formulation. In the case or water-soluble substances which are located in the internal volumes of the liposomes it must be expected that even the addition of water will bring about losses - particularly in the case of low molecular weight substances.

The liposomes available until now have significant disadvantages in spite of the properties mentioned above:
1. On account of the high purity materials usually employed - primarily highly refined phosphatidylcholines - and the complicated method of preparation used liposomes of classical composition are appreciably more expensive than normal emulsions with less favourable properties.
2. Liposomes of classical composition only possess a limited storage capacity for lipophilic substances. Liposomes formed from unsaturated phospholipids may be able to incorporate ca. 10 to 30% of their weight of triglycerides, but even for a highly concentrated liposome dispersion containing 10% liposome component (in the dry substance) this still means a final concentration of only 1 to 3% triglyceride in the complete formulation. In contrast 10 to 20% lipophilic component concentrations are normal for comparable oil-in-water emulsions.

The object of this invention is, therefore, to provide an aqueous dispersion of vesicles whose phospholipid membrane walls are particularly suitable for loading with lipophilic substances.

This object is achieved by an aqueous vesicle dispersion comprising phospholipid vesicles, water and, if necessary, inorganic and/or organic electrolytes characterized in that the membranes of the vesicles consist of a mixture of 10 to 40 parts by weight phosphatidylcholine, 30 to 80 parts by weight of a lipophilic substance and 10 to 30 parts by weight of a phospholipid base substance and that this has the following composition:
15 to 25% by weight phosphatidylethanolamine,
15 to 25% by weight phosphatidylinositol,
15 to 25% by weight phosphatidic acid,
15 to 0% by weight phosphatidylcholine,
0 to 1% by weight oil,
40 to 24% by weight accompanying substances normally found in lecithin,
where the sum of the total amounts is 100% and the vesicle dispersion comprises 35.0 to 99.7% by weight water as a proportion of the total weight.

To this dispersion can also be added 0.1 to 5% by weight of an inorganic base and/or organic base and 0.1 to 5% by weight of an inorganlc and/or organic electrolyte in order to adjust the complete mixture to a physiological pH and a physiological osmolarity.

For good physiological compatibility the pH value is adjusted to the 5 to 8 range, preferably between 6.5 and 7. The osmolarity range is 150 to 450 m osmol/L and preferably 250 to 350 m osmol/L.

Lipophilic substances are particularly well retained by the vesicle membranes. Thus the membranes can be loaded with vitamin E, retinoids, steroids, lipophilic and amphiphilic active substances, vegetable and animal oils, radical scavengers and UV absorbers. Oils are particularly favoured lipophilic substances.

In particular lipophilic active substances, vegetable and animal oils are important for optimal skin care in the cosmetic field, especially for the treatment of dry skin. In the case of polyunsaturated oils, such as are used for the treatment of atopic dermatitis (H.P. Nissen, W. Wehrmann, U. Kroll and H.W. Kreysel, Fat. Sci. Technol. 90 (7), 268-271 (1988)), the distribution and penetration of the skin is of decisive importance. For this reason liposomes are the ideal carrier system for distribution and penetration of the skin.

The greater loading of the phospholipid membrane walls also tends to favour the uptake of greater concentrations of triglycerides.

The substantial loading of the membranes makes it possible to employ the vesicle dispersions according to the invention for pharmaceutical and cosmetic formulations. These can not only incorporate active ingredients but also the additives commonly employed in such compositions.

According to the invention the vesicle dispersion comprising aqueous phospholipid vesicles, water and any inorganic and/or organic electrolytes is prepared by dispersing the phospholipids in water. The procedure is as follows: 0.1 to 20 parts by weight of a phospholipid base of the following composition:
15 to 25% by weight phosphatidylethanolamine,
15 to 25% by weight phosphatidylinositol,
15 to 25% by weight phosphatidic acid,
15 to 0% by weight phosphatidylcholine,
0 to 1% by weight oil,
40 to 24% by weight accompanying substances normally found in lecithin
is dispersed in 99.7 to 35 parts by weight water at a temperature between 10°C and 80°C, 0.1 to 20 parts by weight phosphatidylcholine are incorporated, 0.1 to 25 parts by weight of a lipophilic substance are added and, if necessary, the pH value of the dispersion is adjusted to a value between 5 and 8 by the addition of inorganic or organic base, if necessary, the dispersion is adjusted to the desired osmolarity by the addition of an inorganic and/or organic electrolyte and then further homogenized whereby the total time of homogenization is 5 to 60 minutes.

In the method according to the invention the phosphatidylcholine can be added as the pure substance, in the form of an oil-containing fraction or in a component that also contains oil-containing or lipophilic materials.

The basic substance consists of a phospholipid mixture containing a particularly high proportion of accompanying phospholipids (with respect to phosphatidylcholine) and which can have the following composition:
15 to 25% by weight phosphatidylethanolamine,
15 to 25% by weight phosphatidylinositol,
15 to 25% by weight phosphatidic acid,
15 to 0% by weight phosphatidylcholine,
0 to 1% by weight oil,
40 to 24% by weight accompanying substances normally found in lecithin,
whereby the total sum amounts to 100% by weight for each composition.

The basic substance is a granulatable solid of colourless to pale beige appearance manufactured by the extraction of raw lecithin with ethanol after which the extraction residue is subjected to the deoiling process usual for raw lecithin (cf. H. Pardun, Die Pflanzenlecithine. Verlag für chemische Industrie, Augsburg 1988) and hence is very economically priced.

On account of the "natural starting material" the composition of the basic substance is subject to variations which can affect the pH and osmolarity of the dispersions produced. It is, therefore, appropriate to adjust the pH range of the dispersion being optimal for phospholipids to between 6 and 7, preferably 6.5 with a base, for example sodium hydroxide, potassium hydroxide, lithium hydroxide, triethanolamine etc., such as is usually employed in pharmacy or cosmetics and then to adjust to the desired physiological osmolarity in the range of 150 to 450 and preferably 250 to 350 m osmol/L by adding a suitable electrolyte. The preferred electrolytes are alkali metal salts such as sodium chloride, sodium sulphate and other sulphates, chlorides and phosphates. The dispersion can also be brought to the desired osmolarity with the aid of a conventional citrate or phosphate buffer.

Naturally other ionic additives that promote the purpose for which the formulation is intended and that are compatible with the formulation can also be used. Particularly in the manufacture of cosmetic preparations, for instance, salts of lactic acid and of pyrrolidone carboxylic acid can be incorporated as components of the natural moisturizing factor.

The temperature of preparation of the dispersion has little effect on the physical properties of the formulation. It is, therefore, convenient to work at room temperature or in the temperature range of 10 to 80°C. This means that temperatures in the range of 70 to 80°C, usual for the reduction of microbial contamination, can be employed. If desired the system can also be processed at lower or higher temperatures.

The process also possesses the additional great advantage that there is no need to add any of the conventional additives for vesicle preparation, e.g., cholesterol, glycerol, dicetyl phosphate, and only physiologically compatible components are required.

The proportions of basic substance, of phosphatidylcholine and oil (or of any other cosmetically, pharmaceutically or technically interesting lipophilic substance) can be varied within the following limits:

| | |
|---|---|
| Basic substance | 0.1 to 20.0% |
| Phosphatidylcholine | 0.0 to 20.0% |
| Lipophilic substance, e.g., oil | 0.0 to 25.0% |

It is evident from these figures that the basic substance alone can produce vesicles and can be combined with lipophilic substances without the addition of phosphatidylcholine. On the other hand given proportions of basic substance and phosphatidylcholine correspond to a maximum amount of lipophilic substance (oil), of which as high a proportion as possible is the aim of this invention. Furthermore it has been found that it is not absolutely necessary to add phosphatidylcholine as the pure substance since it can be added in the form of "fractions" i.e., in enriched ford too. Such "fractions" are commercially available and contain large proportions of oil (25 to 75%). This oil component is usually soy, sunflower, thistle or rape oil. On the other hand "compounds" of phosphatidylcholine and oils or "fractions" and oils are also applied since they are easier to handle.

The cosmetic formulations so produced combine the excellent skin-care properties of the phospholipids (lecithins) and of the native oils or other lipophilic agents conventionally used in cosmetics with simultaneous excellent distribution and skin penetration (H. Lautenschläger, J. Röding and M. Ghyczy, Seifen, Öle, Fette, Wachse 114 (14), 531 (1988)). Thus the preparations described can be employed successfully for daily facial and body care, particularly for the treatment of dry skin, for the treatment of skin blemishes and for the re-establishment of optimal linoleic acid levels in the deeper layers of the skin.

The basic substance employed in the examples had the following composition
(Source: soybeans):

| | |
|---|---|
| Phosphatidylethanolamine, | 20.2% by weight |
| Phosphatidylinositol, | 19.4% by weight |
| Phosphatidic acid, | 22.0% by weight |
| Phosphatidylcholine, | 10.6% by weight |
| N-acylcephalin | 2.3% by weight |
| Lysolecithin | less than 1.0% by weight |
| Oil | less than 1.0% by weight |
| Other substances normally accompanying lecithin | to 100.0% by weight |

### Example 1

A mixture of 5 g basic substance and 7 g phosphatidylcholine (Phospholipon 90^{R}) is homogenized in 62.14 g water with the aid of a high performance stirrer (e.g., rotor-stator based, dissolver-stirrer or high pressure homogenizer). Then 20 g soybean oil is stirred into the dispersion, the mixture is homogenized once more and then adjusted to physiological osmotic pressure by the addition of 0.73 g sodium chloride and 5 g water with further homogenization. The dispersion is filtered and can - if desired - be treated with a preservative to improve storage.

The mean particle size of the vesicles so formed is 420 nm (measured by the laser scattering method).

This example indicates that it is possible to achieve an oil content of ca. 20% in the complete formulation even with a relatively small proportion of phosphatidylcholine. The oil concentration is six times higher than that of classical liposome concentrates, even though the phosphatidylcholine content is only ca. one half that usually employed.

The dispersion can be prepared using practically all types of stirrers conventionally employed in the manufacture of pharmaceuticals and cosmetics. The vesicles produced are larger or smaller depending on the stirrer employed and the stirring time, which may vary between 5 and 60 minutes. In general the vesicle size lies in the range of 100 to 500 nm but can also lie below 100 nm.

### Example 2

A mixture of 5 g basic substance and 5 g phosphatidylcholine (Phospholipon 90^{R}) is homogeneously dispersed in 80 g water with the aid of a high performance stirrer. The dispersion is then neutralized (pH 7) with a little sodium hydroxide, 15 g evening primrose oil is stirred in and the mixture homogenized once more, its osmolarity is then adjusted to physiological osmolarity with sodium citrate while it is further homogenized. The dispersion is filtered and can - if desired - be treated with a suitable preservative to improve storage.
The mean particle size is 210 nm
(laser scattering method).

### Example 3

A dispersion of 5 g basic substance in 80 g water is prepared by homogenizing with a high efficiency stirrer. Into the dispersion is stirred 15 g of a "compound" of 50% phosphatidylcholine and 50% oil (major component thistle oil; commercial name: Phosal 50 SA^{R}), the mixture is adjusted to pH 6.5 to 7 with a little sodium hydroxide, homogenized again and finally adjusted to a physiological osmolarity by the addition of a little sodium chloride under continued homogenization. The dispersion is filtered and can - if desired - be treated with a suitable preservative to improve storage.
The mean particle size is 251 nm
(laser scattering method).

Naturally in the method described in Example 3 the "compound" can be diluted with thistle oil, other oils or lipophilic cosmetic or pharmaceutical active ingredients before addition to the basic substance dispersion so as to achieve other desired oil or active ingredient concentrations. The dispersions so prepared can be used directly for the manufacture of pharmaceutical, cosmetic and technical products as illustrated in the following example of a cosmetic skin care formulation.

### Example 4

A 100 g sample of the dispersion obtained in Example 1 is placed in vacuum and treated with 0.5 g xanthan gum (Rhodigel 200^{R}) with vigorous stirring, when the viscosity is greatly increased and further stirring yields the completed skin care product.

Naturally in the case of the process described in Example 4 all the usual compatible cosmetic additives and active ingredients such as antioxidants, preservatives, gel formers, consistency providers, perfumes, vitamins etc. can be added to and worked into the product according to the methods known to the expert in the art.

Example 5 illustrates the typical preparation of a liposome creme:

### Example 5

A high performance stirrer is employed to disperse 5 g basic substance homogeneously in 64.6 g water. Homogenization is continued while 7 g phosphatidylcholine (Phospholipon 90^{R}), 10 g jojoba oil (Dragoco) and 0.2 g vitamin E acetate (Rhône-Poulenc) are added in succession. The dispersion is buffered with a mixture of 0.7 g potassium dihydrogen phosphate, 0.9 g disodium hydrogen phosphate dodecahydrate (Na₂HPO₄·12 H₂O) and 5 g water and briefly homogenized once again; this adjusts the pH to 6.5. The vesicle size is 493 nm, the osmolarity 267 m osmol. Then a solution of 5 g propylene glycol and 0.5 g phenonip (NIPA) is added as preservative, 1 g xanthan gum (Rhodigel 200^{R}) is added as thickening agent and 0.1 g perfume oil as perfume agent. Finally the complete mixture is homogenized once more and the product is filled into tubes.

## Claims

1. Aqueous vesicle dispersion comprising phospholipid vesicles, water and, if necessary, inorganic and/or organic electrolytes, characterized in that the membranes of the vesicle consist of a mixture of 10 to 30% by weight of a phospholipid base substance with the following composition:
15 to 25% by weight phosphatidylethanolamine,
15 to 25% by weight phosphatidylinositol,
15 to 25% by weight phosphatidic acid,
15 to 0% by weight phosphatidylcholine,
0 to 1% by weight oil,
40 to 24% by weight accompanying substances normally found in lecithin,
where the sum of the total amounts is 100% by weight in each case,
10 to 40% by weight phosphatidylcholine and
30 to 80% by weight of a lipophilic substance
and the vesicle dispersion contains 35.0 to 99.7% by weight water as proportion of the total weight.

2. Aqueous vesicle dispersion according to Claim 1 characterized in that the vesicle dispersion contains
0.1 to 5% by weight of an inorganic and/or organic base and/or
0.1 to 5% by weight of an inorganic and/or organic electrolyte.

3. Aqueous vesicle dispersion according to Claim 1 characterized in that the lipophilic substance is an oil.

4. Method for the preparation of an aqueous vesicle dispersion according to any of Claims 1 to 3, by dispersing the vesicle-forming substance in water characterized by dispersing 0.1 to 20 parts by weight of a phospholipid basic substance of the following composition:
15 to 25% by weight phosphatidylethanolamine,
15 to 25% by weight phosphatidylinositol,
15 to 25% by weight phosphatidic acid,
15 to 0% by weight phosphatidylcholine,
0 to 1% by weight oil,
40 to 24% by weight accompanying substances normally found in lecithin,
at a temperature between 10°C and 80°C in 99.7 to 35 parts by weight water, with the addition of 0.1 to 20 parts by weight phosphatidylcholine, the incorporation of 0.1 to 25 parts by weight of a lipophilic substance, if necessary the adjustment of the pH of the dispersion with an inorganic or organic base to a value between 5 and 8 and if necessary adjustment of the desired osmolarity by addition of an inorganic and/or organic electrolyte followed by homogenization by stirring with a total homogenization time of 5 to 60 minutes.

5. A method for the preparation of a vesicle dispersion according to Claim 4 characterized in that the phosphatidylcholine is added as pure substance, in the form of an oil-containing fraction or as a component with oil-containing or lipophilic substances in addition to phosphatidylcholine.

6. Use of the aqueous vesicle dispersion according to any of Claims 1 to 5 for the preparation of pharmaceutical or cosmetic compositions characterized in that additives are added to these compositions, if necessary.

7. Use according to Claim 6 for the manufacture of cosmetic compositions for skin care.

## Patentansprüche

1. Wäßrige Vesikeldispersion, enthaltend Phospholipidvesikel, Wasser und ggf. anorganische und/oder organische Elektrolyte, dadurch gekennzeichnet, daß die Membranen der Vesikel aus einer Mischung von
10 bis 30 Gew.% eines Phospholipidgrundstoffes, der die nachstehend wiedergegebene Zusammensetzung aufweist:
15 bis 25 Gew.% Phosphatidylethanolamin,
15 bis 25 Gew.% Phosphatidylinositol,
15 bis 25 Gew.% Phosphatidsäure,
15 bis 0 Gew.% Phosphatidylcholin,
0 bis 1 Gew.% Öl, und
40 bis 24 Gew.% üblicher Lecithin-Begleitstoffe,
wobei sich die Mengen auf jeweils 100 Gew.% summieren,
10 bis 40 Gew.% Phosphatidylcholin und
30 bis 80 Gew.% eines lipophilen Stoffes bestehen,
und daß die Vesikeldispersion 35 Gew.% bis 99,7 Gew.% Wasser, bezogen auf das Gesamtgewicht, enthält.

2. Wäßrige Vesikeldispersion nach Anspruch 1, dadurch gekennzeichnet, daß die Vesikeldispersion 0,1 Gew.% bis 5 Gew.% einer anorganischen und/oder organischen Base und/oder 0,1 Gew.% bis 5 Gew.% eines anorganischen und/oder organischen Electrolyten enthält.

3. Wäßrige Dispersion nach Anspruch 1, dadurch gekennzeichnet, daß der lipophile Stoff ein Öl ist.

4. Verfahren zur Herstellung einer wäßrigen Vesikeldispersion nach einem der Ansprüche 1 bis 3 durch Dispergieren der die Vesikel ausbildenden Substanz in Wasser, gekennzeichnet durch, Dispergieren von 0,1 Gewichtsteilen bis 20 Gewichtsteilen eines Phospholipidgrundstoffes, der die nachfolgend wiedergegebene Zusammensetzung aufweist:
15 bis 25 Gew.% Phosphatidylethanolamin,
15 bis 25 Gew.% Phosphatidylinositol,
15 bis 25 Gew.% Phosphatidsäure,
15 bis 0 Gew.% Phosphatidylcholin,
0 bis 1 Gew.% Öl, und
40 bis 24 Gew.% üblicher Lecithin-Begleitstoffe,
bei einer Temperatur zwischen 10 °C und 80 °C in 99,7 bis 35 Gewichsteilen Wasser,
Hinzufügen von 0,1 bis 20 Gewichtsteilen Phosphatidylcholin,
Einbringen von 0,1 bis 25 Gewichtsteilen eines lipophilen Stoffes, ggf. Einstellen des pH-Wertes der Dispersion mit einer anorganischen oder organischen Base auf einen Wert zwischen 5 und 8, und
ggf. Einstellen einer gewünschten Osmolarität durch Zusatz eines anorganischen und/oder organischen Elektrolyten und anschließender Homogenisierung durch Rühren bei einer Gesamthomogenisierungszeit von 5 Minuten bis 60 Minuten.

5. Verfahren zur Herstellung einer Vesikeldispersion nach Anspruch 4, dadurch gekennzeichnet, daß das Phosphatidylcholin als Reinsubstanz, in Form einer ölhaltigen Fraktion oder als eine solche Komponente hinzugefügt wird, die ölhaltige oder lipophile Stoffe zusätzlich zu dem Phosphatidylcholin enthält.

6. Verwendung der wäßrigen Vesikeldispersion nach einem der Ansprüche 1 bis 5 zur Herstellung von pharmazeutischen oder kosmetischen Zusammensetzungen, dadurch gekennzeichnet, daß zu diesen Zusammensetzungen ggf. Hilfsstoffe hinzugefügt werden.

7. Verwendung nach Anspruch 6 für die Herstellung von kosmetischen Zusammensetzungen zur Hautpflege.

## Revendications

1. Dispersion aqueuse de vésicules, qui comprend des vésicules de phospholipides, de l'eau et, si cela se révèle nécessaire, des électrolytes inorganiques et/ou organiques, caractérisée en ce que les membranes des vésicules sont constituées d'un mélange de 10 à 30% en poids d'une substance basique phospholipidique, présentant la composition suivante :
15 à 25% en poids de phosphatidyléthanolamine,
15 à 25% en poids de phosphatidylinositol,
15 à 25% en poids d'acide phosphatidique,
15 à 0% en poids de phosphatidylcholine,
0 à 1% en poids d'huile,
40 à 24% en poids de substances d'accompagnement qui se
trouvent normalement dans la lécithine, où la somme des proportions totales forme 100% en poids dans chaque cas,
10 à 40% en poids de phosphatidylcholine et
30 à 80% en poids d'une substance lipophile
et la dispersion de vésicules contient 35,0 à 99,7% en poids d'eau en proportion du poids total.

2. Dispersion aqueuse de vésicules suivant la revendication 1, caractérisée en ce que cette dispersion de vésicules contient
0,1 à 5% en poids d'une base inorganique et/ou organique et/ou
0,1 à 5% en poids d'un électrolyte inorganique et/ou organique

3. Dispersion aqueuse de vésicules suivant la revendication 1, caractérisée en ce que la substance lipophile est une huile.

4. Procédé de fabrication d'une dispersion aqueuse de vésicules suivant l'une quelconque des revendications 1 à 3, par la dispersion d'une substance formatrice de vésicules dans de l'eau, caractérisé en ce que l'on disperse 0,1 à 20 parties en poids d'une substance basique phospholipidique présentant la composition suivante :
15 à 25% en poids de phosphatidyléthanolamine,
15 à 25% en poids de phosphatidylinositol,
15 à 25% en poids d'acide phosphatidique,
15 à 0% en poids de phosphatidylcholine,
0 à 1% en poids d'huile,
40 à 24% en poids de substances d'accompagnement qui se
trouvent normalement dans la lécithine, à une température comprise entre 10°C et 80°C, dans 99,7 à 35 parties en poids d'eau, en procédant à l'addition de 0,1 à 20 parties en poids de phosphatidylcholine, l'incorporation de 0,1 à 25 parties en poids d'une substance lipophile, en recourant, si cela se révèle nécessaire, à l'ajustement du pH de la dispersion à l'aide d'une base inorganique ou organique jusqu'à une valeur comprise entre 5 et 8 et, si cela se révèle nécessaire, à l'ajustement de l'osmolarité souhaitée par l'addition d'un électrolyte inorganique et/ou organique, suivie de l'homogénéisation par agitation pendant une période d'homogénéisation totale de 5 à 60 minutes.

5. Procédé de fabrication d'une dispersion de vésicules suivant la revendication 4, caractérisé en ce que l'on ajoute la phosphatidylcholine sous forme de substance pure, sous forme d'une fraction contenant de l'huile, ou sous forme d'un composant avec des substances lipophiles ou contenant de l'huile, en plus de la phosphatidylcholine.

6. Utilisation de la dispersion aqueuse de vésicules suivant l'une quelconque des revendications 1 à 5 en vue de la fabrication de compositions pharmaceutiques ou cosmétiques, caractérisée en ce que l'on ajoute des additifs à ces compositions si cela se révèle nécessaire.

7. Utilisation suivant la revendication 6 en vue de la fabrication de compositions cosmétiques destinées au soins de la peau.
